(19) Europäisches Patentamt

European Patent Office

Office européen des brevets

(11) Veröffentlichungsnummer: **0 286 847 B1**

(12) **EUROPÄISCHE PATENTSCHRIFT**

(45) Veröffentlichungstag der Patentschrift: **02.11.94**

(21) Anmeldenummer: **88104015.8**

(22) Anmeldetag: **14.03.88**

(51) Int. Cl.⁵: **A61K 39/00**

(54) Verwendung antigener Substanzen zur Prophylaxe oder Therapie von Störungen und Krankheiten im Verdauungstrakt von Tieren und Menschen.

(30) Priorität: **15.04.87 DE 3712890**

(43) Veröffentlichungstag der Anmeldung:
**19.10.88 Patentblatt 88/42**

(45) Bekanntmachung des Hinweises auf die
Patenterteilung:
**02.11.94 Patentblatt 94/44**

(84) Benannte Vertragsstaaten:
**AT BE CH DE ES FR GB IT LI NL SE**

(56) Entgegenhaltungen:
**DE-A- 3 132 412**
**DE-A- 3 733 899**
**FR-A- 2 408 351**
**GB-A- 2 008 404**
**GB-A- 2 132 466**

**VETERINARY RECORD, vol. 92, 1973; pp. 630-636&NUM;**

**VETERINARY RECORD, vol. 97, 1975; pp. 24-28&NUM;**

(73) Patentinhaber: **EFFEM GmbH**
**Eitzer Landstrasse 215**
**D-27283 Verden (DE)**

(72) Erfinder: **Teichmann, Reinhard K., Dr. med. habil**
**Drosselweg 81**
**D-7417 Pfullingen (DE)**
Erfinder: **Brendel, Walter, Prof. Dr. med., Dr. h.c.**
**verstorben**
**(DE)**
Erfinder: **Liebich, Hans-Georg, Prof.Dr. vet. med.**
**Germaniastrasse 5**
**D-8000 München 40 (DE)**

(74) Vertreter: **Goddar, Heinz J., Dr. et al**
**FORRESTER & BOEHMERT**
**Franz-Joseph-Strasse 38**
**D-80801 München (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung bezieht sich auf die Verwendung von mindestens einer antigenen Substanz zur Herstellung eines Medikamentes zur Prophylaxe und Therapie von Störungen und Krankheiten im Verdauungstrakt von Tieren und Menschen.

Krankhafte Störungen im Verdauungstrakt von Tieren und Menschen stellen weltweit ein ernstes, wirtschaftlich bedeutendes Problem dar. Im humanmedizinischen Bereich ist beispielsweise die Ulcuskrankheit unter den chronischen Krankheiten mit einer lebenslänglichen Prävalenz von etwa 8 bis 10 Prozent in Europa und Nordamerika vorherrschend. Beispielsweise betrungen die Gesamtausgaben für Ulcuskranke in den Ländern USA, den Niederlanden, Italien und Schweden einschließlich des Arbeitsausfalles etwa 1 Prozent der jeweiligen, nationalen, jährlichen Ausgaben im Gesundheitswesen. In Deutschland ist weiterhin beispielsweise die Sterblichkeit wegen Ulcusleiden in der Zeit von 1952 bis 1980 nur unwesentlich von 7 auf 6 Todesfälle pro 100.000 Einwohner zurückgegangen (Sonnenberg, A., Fritsch, A.: Changing mortality of peptic ulcer disease in Germany. Gastroenterology 84, 1553 (1983)). Innerhalb dieses Zeitraumes hat sich jedoch das Ulcusleiden als tödliche Krankheit der mittleren und älteren Jahresgruppe verschoben, hin zu einer tödlichen Erkrankung vornehmlich des Alters und hohen Alters. Die Sterblichkeit wegen Magen- und Duodenalulcus bei Frauen hat dabei zugenommen. Die Sterblichkeit bei Männern bezüglich des Duodenalulcus blieb konstant und diejenige des Magenulcus nahm etwas ab.

Bei der Ulcustherapie werden derzeit vorwiegend H2-Antagonisten eingesetzt (Bauernfeind et al., Ulcustherapie, JAMA, Heft 2, 135 (1986)). Das Prinzip der H2-Antagonisten-Therapie besteht in der Verringerung der aggressiven Säurewirkung im Verdauungstrakt und ist, wie aus der genannten Veröffentlichung ersichtlich ist, mit einer Reihe unangenehmer Nebenwirkungen behaftet. Ein wesentliches Problem bei Ulcuserkrankungen stellt weiterhin die Verhütung eines Ulcusrezidivs dar, da, wie bereits erwähnt, die Ulcuserkrankung zu den chronischen Krankheiten mit lebenslanger Prävalenz zählt. Beispielsweise ergab eine Drei-Jahres-Langzeit- studie an 44 Zentren in 13 Ländern mit insgesamt 1.423 Patienten lediglich eine Rezidivfreiheit von 81 Prozent nach 12 Monaten, 73 Prozent nach 24 Monaten und 65 Prozent nach 36 Monaten für den Duodenalulcus (DMW 111.Jg.Nr.3, 117, (1986)). Nach alledem ergibt sich die dringende Notwendigkeit nach alternativen, nebenwirkungsfreien Medikamenten zur Ulcusverhinderung oder -behandlung, die insbesondere auf lange Dauer und prophylaktisch angewendet werden kann.

Nicht nur im humanmedizinischen Bereich, sondern auch in der Veterinärmedizin, und hier insbesondere in der Schweine-, Rinder- und Geflügelzucht, stellen Diarrhöe und Ulcuserkrankungen ein gravierendes Problem dar. Es ist beispielsweise ein bekanntes Phänomen, daß Jungschweine nach Absetzen vom Muttertier und übergang zu breiflüssigem Kraftfutter Ulcera erleiden, an denen sie zum Teil sterben. In der EG stehen 120 bis 160 Millionen Schweine (geschätzte Werte), von denen 3 bis 4 Prozent erkranken. Geht man aufgrund der Erkrankungen von einem Schweineverlust von etwa 4 Millionen pro Jahr aus, ergibt sich ein finanzieller Verlust von bis zu 1,2 Milliarden DM pro Jahr. Die Notwendigkeit einer wirksamen Bekämpfung dieser Störungen liegt auf der Hand. Ähnliches gilt für die Rinder- und Geflügelzucht.

Aus der GB-A-2008404 ist ein Verfahren zur Verhinderung von Diarrhöe als Folge einer kozidien Infektion bei Geflügel durch Aufzucht mit einem Futter, das zugesetzte, lebensfähige, sporolierte Urozysten wenigstens einer Kokzidienart enthält, für die das Geflügel anfällig ist, wobei die Oozysten in einer Konzentration vorhanden sind, die nur ausreichend ist, um eine subklinische Infektion im Geflügel zu induzieren.

Aus the Veterinary Record 92,630-636 (1973) ist ein Verfahren zur Prophylaxe von Darmkrankheiten bei Schweine durch orale Immunisierung und weitere orale Gabe von hitze-inaktivierten Antigen-Zubereitungen von E. coli-Stämmen, welche zu Darmkrankheiten führen, bekannt.

Aus the Veterinary Record 97,24-28 (1975) ist schließlich noch eine Verfahren zur Prophylaxe und Therapie von Darmkrankheiten bei präruminanten Kälbern durch orale Immunisierung und weitere orale Gabe von hitze-inaktivierten Antigen-Zubereitungen von E. coli- und Salmonella- Stämmen, welche zu Darmkrankheiten führen, bekannt.

Für die Pathogenese von Ulcusleiden scheinen aggressive Faktoren im Verdauungsvorgang, beispielsweise die Sekretion von Säure und Pepsin, eine wesentliche Bedeutung zu haben (Grossman, M.I.: Regulation of gastic acid secretion. In: Physiology of the Gastrointestinal Tract. Ed.: L.R. Johnson.Raven Press, New York.659-671 (1981)).

Diesen aggressiven Faktoren stehen protektive physiologische Verdauungsmechanismen gegenüber. Zu diesen protektiven Mechanismen gehört die Schleimbildung im Magen, die, zusammen mit der Bikarbonatsekretion als erste Schicht einer Mucosabarriere angesehen wird.

Die verschiedenen Sekretionsleistungen des Magens sowie die Zellregeneration unterliegen neurohumoralen Kontrollen. Das Hormon, das die genannten aggressiven Verdauungsleistungen in Gang setzt, ist das Gastrin, das 1964 isoliert wurde (Gregory, R.A., Tracy, H.J.: The constitution and properties of two gastrins extracted from hog antral mucosa. Gut 5, 103-117, (1964)).

Bei den Untersuchungen über die Stimulierung der gastralen Funktionen ist bis vor kurzem ein Aspekt völlig unberücksichtigt geblieben: Mit jeder Nahrungsaufnahme kommt die Schleimhaut des Magens auch in Kontakt mit Substanzen, die als Antigene angesehen werden können. Obwohl es bereits Hinweise für immunologische Strukturen im Magen gab, fehlte dennoch bis dahin jegliche Vorstellung über deren Funktion. Anhand von experimentellen Befunden ist nunmehr kürzlich festgestellt worden, daß nach vorausgegangener Immunisierung mit eines Antigen bei wiederholtem Kontakt des Verdauungstrakts mit diesem Antigen im Antrum des Magens eine Immunreaktion abläuft, welche die gesamte gastrale Verdauungskaskade in Gang setzt. Diese besteht im wesentlichen in einer Erhöhung der Magendurchblutung im Antrum und Duodenum, der Schleimbildung des Magens sowie einer Steigerung der Gastrinproduktion. Der Nachweis einer immunologischen Stimulierung gastraler Verdauungsmechanismen beitet eine völlig neue Sicht der Physiologie und Pathophysiologie der Verdauungstätigkeit. Die Erkenntnisse wurden am Tiemodell gewonnen, bei dem nach vorausgangener Immunisierung luminale Antigene im Magen erkannt wurden und Verdauungsprozesse induzierten (Teichmann, R.K., Andress, H.J., Gycha, S., Seifert J., Brendel, W.: Immunologic mediated gastrin release. Gastroenterology **84,2** (1983)).

Immunfluoreszenzmikroskopische Untersuchungen zeigten bei sensibilisierten Tieren außerdem in der Lamina propria mucosae Bindungsstellen für das luminal verabreichte Antigen. Ferner fanden sich im Antrum nach Antigengabe vermehrt Zellen mit immunassoziierten Antigenen auf ihrer Oberfläche, sogenannten Ia-Antigenen. Durch Doppelimmunfluoreszenz ließ sich nachweisen, daß die Bindungsstellen für das luminal verabreichte Antigen auf Ia-Antigen exprimierenden Zellen zu liegen scheinen.

Zudem fand sich bei sensibilisierten Tieren nach Antigengabe ein Anstieg von Plasma- und Mastzellen in der Lamina propria mucosae mit einer zunehmenden Mastzelldegranulation, sowie einem gehäuften Vorkommen intraepithelial gelegener, lymphoider Zellen. (Teichmann, R.K,, Andress, H.J., Liebich,H., Seifert, J.,Brendel,W.: Die Bedeutung immunkompetenter Zellen im Antrum bei der Stimulation von Verdauungsprozessen. Langenbecks Arch.Chir.Suppl. 151, (1984)).

Im Rahmen der immunologisch vermittelten Stimulation gastraler Verdauungsprozesse werden damit neben aggressiven Faktoren wie Säure auch zytoprotektive Mechanismen in Gang gesetzt wie Durchblutungssteigerung und Schleimproduktion.

Basierend auf diesen Ergebnissen wurden weiterführende Untersuchungen über Mediatoren der immunologischen Stimulation gastraler Funktionen am Modell der isolierten Antrum-Gefäßperfusion von Hunden durchgeführt. Im venösen Effluat erfolgten Gastrin- und Prostaglandin $E_2$- und $F_2$-Bestimmungen.

Überstand von Ductus-thoracicus-Lymphe, gesammelt nach Antigengabe bei sensibilisierten Tieren stimulierte bei der isolierten Antrumgefäßperfusion die Gastrinfreisetzung. Diese Aktivität fehlte in der Lymphe bei Verfütterung desselben Antigens an nicht-immunisierte Tiere (Teichmann, R.K., Pratschke, E., Grab, J., Tutert, J., Enders, G., Brendel, W.: Gastrinstimulierende Wirkung von Ductus-thoracicus-Lymphe nach gastrointestinaler Immunreaktion. Langenbecks Arch. Chir. Suppl. 271 (1985)). Daduch wurde gezeigt, daß lösliche Mediatoren für die Antigen-induzierte Gastrinfreisetzung verantwortlich sind.

Die Mukosamastellprodukte Histamin und Leukotrien $C_4$, die bei immunologischen Reaktionen freigesetzt werden, hemmen signifikant die antrale Gastrinfreisetzung. Gastrin selbst stimuliert die Histaminfreisetzung, so daß diese Ergebnisse erstmals Hinweise für einen lokal im Antrum befindlichen, negativen Rückkopplungsmechanismus zwischen G-Zelle und Mastzelle geben (Pratschke, E., Teichmann, R.K., Grab, J., Tutert, E., Brendel, W.: Der Einfluß von Mastzellprodukten auf die Gastrinfreisetzung. Langenbecks Arch. Chir.Suppl., 295, (1985)).

Parallel zur Hemmung der Gastrinfreisetzung durch Histamin und Leukotrien $C_4$ kommt es zu einer Freisetzung der endogenen Prostaglandine $E_2$ und $F_2$. Vom Prostaglandin $E_2$ konnte am Modell der isolierten Antrumperfusion gezeigt werden, daß es die Gastrinfreisetzung hemmt. Dies könnte bedeuten, daß die gastrinhemmende Wirkung von Mukosamastzellmediatoren möglicherweise unter Vermittlung von endogen freigesetzten Prostaglandinen zustande kommt (Pratschke, E., Teichmann, R.K., Grab, J., Enders, G., Brendel, W.: Endogenes Prostaglandin $E_2$ als Regulator der Gastrinfreisetzung. In: Kongreßbericht der 26. Jahrestagung der Österr. Gesellschaft für Chirurgie. Hrsg.: F.Helmer, E. Horcher, Styria-Verlag, Graz, 131, (1985)).

Die im Rahmen von Antigenerkennungsprozessen unter Beteiligung antigenpräsentierender Zellen freiwerdenden Lymphokine Interleukin 2 und Interferon-gamma setzen Gastrin frei. Damit gelang erstmals der Nachweis einer gastrinstimulierenden Wirkung von Mediatoren immunkompetenter Zellen (Pratschke, E., Teichmann, R.K., Grab, J., Hammer, C., Brendel, W.: Gastrinfreisetzung durch Mediatoren immunkompetenter Zellen. Langenbecks Arch. Chir.Suppl., 261 (1986)).

EP 0 286 847 B1

Anhand immunfluoreszenzmikroskopischer Studien der mit Interleukin 2 und Gamma-Interferon perfundierten Antren mit monoklonalen Antikörpern gegen Klasse 2-Antigene des MHC-Komplexes konnte weiterhin gezeigt werden, daß unter der Wirkung von Interferon-gamma antigene Strukturen der DLA-Klasse 2 (Dog-Leukocyte-Antigens) auf Epithelzellen und Makrophagen, wahrscheinlich auch auf intraepithelialen T-Lymphozyten exprimiert werden, jedoch nicht unter Interleukin 2.

Als Mediatoren dieser Immunreaktion kommen somit beispielsweise Interleukin 2, aber auch Prostaglandine und andere Substanzen in Frage.

Die geschilderten Antigen-induzierten Reaktionen werden somit für die Entstehung von Ulcera diskutiert und sind möglicherweise aber auch für den gesamten Gastrointestinaltrakt von Bedeutung. Wenn Antigen-induzierte Steigerung der Antrum-Durchblutung, der Gastrin- und Schleimsekretion ausbleibt oder lokal gestört ist, kann die enstehende Säure nicht ausreichend gepuffert und beim Ausbleiben der Durchblutungssteigerung nicht abgeführt werden, so daß die Säure in die Mucosa eindringt und dort möglicherweise lokale Ulcera auslöst.

Im Rahmen der Untersuchungen über die immunologische Stimulierung der gastralen Reaktionen wurde festgestellt, daß nach Immunstimulierung mit einem synthetischen Antigen neben der Antigen-spezifisch induzierten Freisetzung von Gastrin als dem stärksten Stimulator der gastralen Säureproduktion im Sinne einer Steigerung der aggressiven Verdauungsleistungen, auch die antrale Sekretion von Somatostatin, das über die Hemmung von Säure- und Pepsinogensekretion protektiv wirksam ist, erhöht werden kann (Krämling, H.-J., Teichmann R.K., Merkle, R., Enders, G., Brendel, W.: Gastrate Protektion durch immunologisch induzierte Somatostatinfreisetzung, 153. Tagung Vereinigung Niederrheinisch-Westfälischer Chirurgen, Köln, 9.-11.Okt.1986).

Die geschilderten Erkenntnisse führen zu vollständig neuen, umfassenden und bisher noch nicht erkannten Möglichkeiten der gezielten Einflußnahme auf Verdauungsvorgänge in protektivem Sinn hinsichtlich aller denkbaren Störungen und Krankheiten im Verdauungstrakt. Von größtem Interesse sind dabei im humanmedizinischen Bereich Erkrankungen wie Erosionen, Ulcera und nicht zuletzt Karzinome. Im veterinärmedizinischen Bereich ist in erster Linie die kritische Übergangsphase von Jungtieren nach Absetzen vom Muttertier und darauffolgendem Kontakt mit Kraftfutter durch die Ausbildung von Erosionen und Ulcera in dieser Phase von Interesse.

Eine im breitesten Sinne anwendbare protektive Induzierung der Funktionen des Gastrointestinaltrakts, basierend auf den geschilderten, fundamentalen Erkenntnissen über die Zusammenhänge immunologischer Funktionen und protektiver physiologischer Verdauungsreaktionen, ergibt sich durch die Verwendung von mindestens einer antigenen Substanz zur Herstellung eines Medikamentes zur Prophylaxe und Therapie von Störungen und Krankheiten im Verdauungstrakt von Tieren und Menschen, wobei nach vorausgegangener Immunisierung bzw. Sensibilisierung durch die antigene(n) Substanz(en) eine nach folgende gastrointestinale Applikation der antigenen Substanz(en) in geeigneter Formulierung erfolgt, durch die protektive physiologische Verdauungsprozesse ausgelöst werden.

Die durch die erfindungsgemäße Verwendung einer antigenen Substanz stimulierten, gastralen Funktionen könnten derart ablaufen daß, nach Sensibilisierung des Magens mit einem in der Nahrung enthaltenen Antigen, bei erneutem Kontakt des Magens mit diesem in der Nahrung enthaltenen Antigen, dieses von sogenannten Antigen-präsentierenden Zellen, beispielsweise Makrophagen, erkannt wird und mit Hilfe von Klasse-2-Antigenen dem T-Lymphozytären System dargeboten wird. Ein Hinweis auf eine Lymphozyten vermittelte immunologische Stimulierung der beschriebenen gastralen Funktionen konnte dadurch erbracht werden, daß immunisierte Hunde mit 80 Rad einer ultraharten Röntgenstrahlung als Ganzkörperbestrahlung ausgesetzt wurden, wodurch, basierend auf der Zerstörung der Lymphozyten, die immunologische Stimulierung der Gastrinfreisetzung gehemmt werden konnte. Die Stimulierbarkeit der Gastrin-Zelle selbst wird durch die Bestrahlung nicht beeinträchtigt.

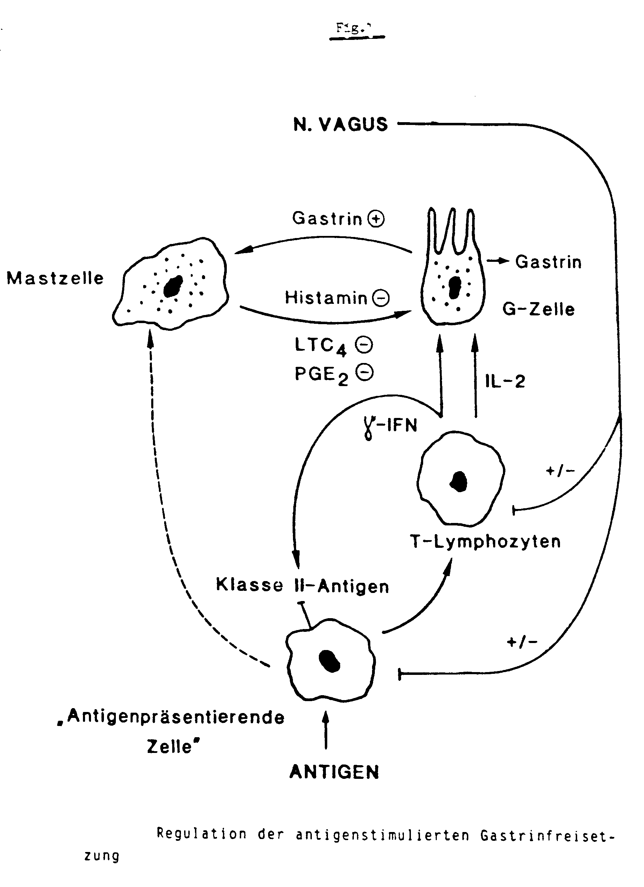

Nach dem Kontakt des auf die geschilderte Weise sensibilisierten Magens mit dem Antigen kommt es zu einer kaskadenartigen Freisetzung von Interleukin 1 und der Stimulation von T-Helfer-Lymphozyten. Die Helferlymphozyten können ihrerseits Interleukin 2 und Interferon-gamma freisetzen, zwei Lymphokine, von denen gezeigt werden konnte, daß sie Gastrin stimulieren. Ein wesentlicher protektiver physiologischer Mechanismus, nämlich die Zunahme der Mucosadurchblutung, die eine von mehreren Stufen der kaskadenartigen protektiven Verdauungsvorgänge darstellt, dürfte durch Mediatoren der Mastzellen, nämlich Histamin, verursacht sein. Neben der Expression von Klasse-2-Antigenen auf Antigen-präsentierenden Zellen des Antrums induziert Interferon-gamma auch die Expression dieser Antigene auf Epithelzellen. Möglicherweise stellt diese zusätzliche "Rekrutierung" der Epithelzellen einen Verstärkermechanismus der Antigenerkennung dar. Der Nervus vagus greift in dieses Antigenerkennungssystem im Sinne einer Immunmodulation ein. Die geschilderten Zusammenhänge sind in Fig. 1 dargestellt.

4

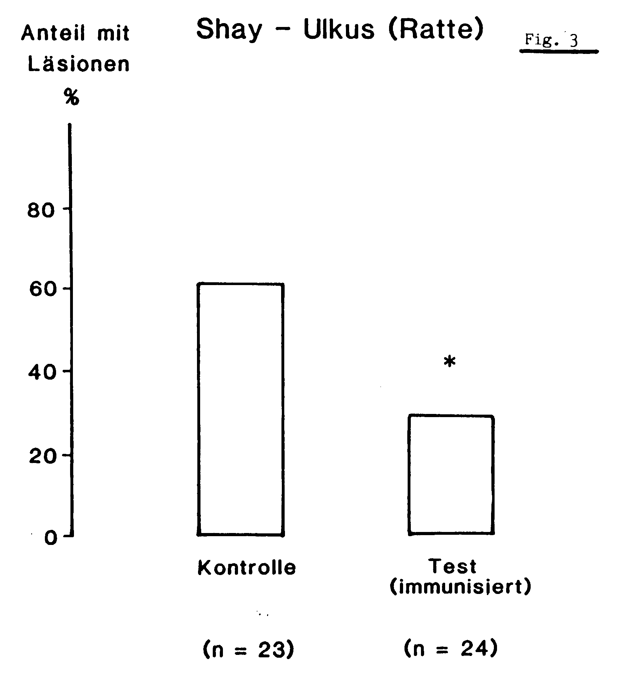

Die Ulcera-protektive Wirkung der Antigen-spezifischen Verdauungsstimulierung wurde am Tiermodell gezeigt. Am Modell des "SHAY-Ulcus" (Shay. H., Komarov, S.A., Fels, S.S., Mekanze, D., Gruenstein, M., Siplet, H.: A simple method for the uniform production of gastric ulceration in the rat. Gastroenterology 5, 43, (1945)), wurde der Einfluß der Antigen-stimulierten Verdauungsregulation geprüft. Männliche Wistar-Ratten wurden dabei mit einem Hapten systemisch immunisiert. Danach erfolgte eine intragastrale Gabe des Haptens sowie als Kontrolle von NaCl. Als weitere Kontrollen wurden nicht-immunsierten Tieren ebenfalls das Hapten bzw. NaCl appliziert. Die so behandelten Ratten wurden nach ihrem Tod, spätestens nach 18 Stunden makroskopisch und mikroskopisch auf gastrale Läsionen untersucht. Dabei zeigt sich eine signifikant geringere Ausprägung bzw. Häufung von Perforationen und großflächigen Wandnekrosen im Vormagenbereich bei den mit dem Hapten immunisierten Tieren (Fig. 3).

Damit konnte am Modell des "SHAY-Ulcus" die protektive Wirkung einer Antigen-spezifischen Verdauungsstimulation nachgewiesen werden. Zwar wird durch die Freisetzung von Gastrin und konsekutiv von Säure auf der Seite aggressiver Faktoren eine ulcerogene Wirkung erzeugt; gleichzeitig werden aber auch protektive Mechanismen stimuliert, beispilsweise Durchblutungserhöhung, Schleimproduktion, möglicherweise über die Freisetzung von Somatostatin, Prostaglandinen und anderen Mediatoren, und dadurch die Ausprägung von schweren Magenmucosa bzw. -wandläsionen verhindert. Diese hier nachgewiesene ulceroprotektive Wirkung der Antigen-induzierten Verdauungsregulation läßt sich im Rahmen der physiologischen Vorgänge teleologisch als "sinnvolle" Steuerung einordnen, da sie gleichzeitig eine Stimulierung der Verdauungsleistung und eine Verminderung des Ulcusrisikos bewirkt. Das Entstehen von Ulcera könnt somit, neben anderen bekannten Ursachen, auch als lokales Versagen der antigenen Reaktionsbereitschaft des Magens verstanden werden. Mit der Stimulierung der gastralen Immunmechanismen durch Sensibilisierung mit Antigenen ergibt sich somit eine gänzlich neue, in größter Breite anzuwendende Möglichkeit zur Prophylaxe und Therapie von Störungen und Krankheiten im Verdauungtrakt von Tieren und Menschen. Der gravierende, weitere Vorteil der Verwendung antigener Substanzen zu dem genannten Zweck, liegt weiterhin in der Tatsache, daß es sich bei den verwendeten Antigenen um biologische Substanzen handelt, mit denen das Säugetier oder der Mensch auch natürlicherweise in Kontakt kommt und das somit keinen, physiologischen Prozessen entgegengerichteten, aggressiven Wirkungsmechanismus hat, sondern vielmehr sinnvolle, protektive physiologische Vorgänge in natürlicher Weise in Gang setzt und somit nebenwirkungsfrei ist.

Im Hinblick auf den geschilderten Mechanismus der Immunstimulierung der Verdauungstätigkeit ist es natürlich von besonderem Vorteil, als antigene Substanzen solche zu verwenden, die in der später zu verdauenden Nahrung enthalten sind. Nach der Sensibilisierung des Tieres oder des Menschen mit der antigenen Substanz wird das Niveau der protektiv wirkenden Faktoren in einer Weise angehoben, die Störungen oder Krankheiten im Verdauungtrakt verhindern oder beheben. Somit sind pflanzliche Eiweiße, vorzugsweise aus Mais oder Soja, oder auch tierische Eiweiße für die Sensibilisierung besonders geeignet, da diese Eiweiße in den von Tier oder Menschen aufgenommenen Futter- oder Nahrungsmitteln häufig vertreten sind.

Als tierisches Eiweiß kann beispielsweise Molkeprotein verwendet werden. Es zeigte sich, daß die prophylaktische Beeinflussung gastraler Schleimhautveränderungen (Erosionen), beispielsweise bei Schweinen, denen während der gesamten Mastperiode bis zur Schlachtung der Schweine nach 10-wöchiger Mastdauer über Trinkflüssigkeit zusätzlich Molkeprotein angeboten wurde, dazu führte, daß insbesondere schwere Vernderungen der Schleimhaut bei den wie beschrieben behandelten Tieren um 70% weniger häufig auftraten als in einer Kontrollgruppe.

Vorzugsweise sollte das Molkeprotein in einer 3 bis 6, besonders bevorzugt 9%igen Molkeproteinlösung vorliegen.

Menschliche Proteine, die Bestandteile von Blut sind und in Blutseren oder anderen Blutderivaten enthalten sein können, beispielsweise Hämoglobin, sind geeignet, um als antigene Substanzen im erfinderischen Sinne verwendet zu werden.

Auch andere Antigene, die nicht zwingenderweise korrespondieren mit Substanzen, die in der aufgenommenen Nahrung enthalten sind, können für die Etablierung der Immunsituation, die für die obengenannten Mechanismen auslösend sein kann, geeignet sein. Beispielsweise wäre im Falle der Immunisierung mit Proteinen, die den Hüllen von Viren entstammen, eine Immunisierung denkbar, die im Sinne der genannten Verdauungsstimulierung wirkt aber auch eine Immunisierung im Hinblick auf die Viren verursachen könnte.

Dasselbe gilt für antigene Substanzen, die in Mikroorganismen, mit denen der Verdauungtrakt in der einen oder anderen Weise konfrontiert wird, enthalten sind. In Rindermägen könnten dies beispielsweise Protozoen sein, im übrigen kann es sich dabei um Bakterien handeln, die bekanntlich stets als Kontaminierung der Nahrung oder dem Futter enthalten sind und somit ebenfalls die obengeschilderten protektiven Prozesse in Gang setzen können. Insbesondere scheinen die Lipopolysaccharide der Zellwand von Escherichia coli, einem natürlichen Darmkommensalen von einigen Säugetieren, geeignet.

Die Verwendung einer antigenen Substanz, die als synthetisch hergestelltes Protein vorliegt, vorzugsweise als kurzes Peptid, das antigene Determinanten für das Auslösen der Immunantwort enthält, ist besonders bevorzugt, da dann eine speziell und gezielt handhabbare Prophylaxe und Therapie möglich ist.

Eine gutgeeignete Substanz für die Induzierung der Immunsituation, aufgrund derer die protektiven Mechanismen anlaufen, sind Haptene, dabei vorzugsweise Nitrophenylessigsäure (NIP). Diese Substanz hat sich im Experiment als besonders gut herausgestellt, um die Immunisierung zu bewirken, die nach nachträglicher intraluminaler Antigen-spezifischer Stimulierung des Magens signifikant die Entstehung von Schleimhautläsionen verhindert. Insbesondere mit dieser Substanz wurde erstmals eine immunologisch vermittelte Ulcusprotektion gezeigt.

Die Verwendung der beschriebenen Nitrophenylessigsäure erfolgt bevorzugt durch Kopplung an ein Trägerprotein, nämlich Ovalbumin.

Wie in der Beschreibung oben geschildert wurde, spielen Immunmodulatoren bei den Gleichgewichtsreaktionen zwischen den aggressiven und protektiven Verdauungsmechanismen eine Rolle. Die Zugabe von Immunmodulatoren zu den antigenen Substanzen kann somit eine für das Auslösen der protektiven Verdauungsmechanismen verstärkende Wirkung haben.

Die Verwendung von Kombinationen von ein oder mehreren der oben geschilderten Substanzen kann eine verbesserte Prohylaxe ode Therapie bewirken.

Die pharmazeutische Konfektionierung einer oder mehrerer der beschriebenen antigenen Substanzen, gegebenenfalls unter Zusatz der Immunmodulatoren, kann sowohl für die enterale als auch parenterale Applikation erfolgen. Im Rahmen der enteralen Konfektionierung können nasal und bronchopulmonal anzuwendende Aerosole eingesetzt werden. Die Konfektionierung für die parenterale Verwendungsweise de antigenen Substanzen betrifft bevorzugt solche zur intramuskulären und/oder subkutanen Verwendung.

Im veterinärmedizinischen Bereich besteht eine Verwendung der antigenen Substanzen darin, die antigene Substanz bei Ferkeln etwa 2 Wochen nach der Geburt zu sensibilisieren. Wie oben bereits erwähnt, erleiden Ferkel nach dem Absetzen vom Muttertier und dem Übergang zu breiflüssigem Kraftfutter Ulcera, an denen sie erkranken oder zum Teil sterben. Bei Behandlung der Ferkel in dieser kritischen Übergangsphase mit Antigenen, die in der später angewendeten Nahrung enthalten sind, können diese Schäden verhindert oder zumindest reduziert werden. Die als antigene Substanzen verwendeten Proteine stellen dabei biologische Substanzen dar, die nach der Applikation völlig rückstandsfrei abgebaut werden können bzw. in nur geringen Mengen als solche ausgeschieden werden.

Eine Möglichkeit der Behandlung der Ferkel besteht darin, daß die Applikation intramuskulär erfolgt, beispielsweise als erste Injektion etwa 2 Wochen nach der Geburt, als zweite Injektion etwa 4 bis 5 Wochen nach der Geburt und als dritte Injektion etwa vor Futterumstellung. Im Hinblick auf die Praktikabilität können die Tiere dann geimpft werden, wenn sie während der Aufzucht vom Züchter ohnehin Eisen-Injectionen erhalten. Die antigenen Substanzen können in einem Kit, der bereits Eisen enthält, zusammengefaßt sein.

Wenn die Jungschweine sodann auf die Nahrung, die diejenigen Proteine enthält, mit denen die Tiere vorher sensibilisiert wurden, umgestellt werden, kann dem Trinkwasser das Antigen zugegeben werden, um die Protektion der Magenschleimhaut durch die beschriebenen Immunmechanismen weiter zu induzieren und zu erhalten.

Im humanmedizinischen Bereich ist die Verwendung der antigenen Substanzen besonders geeignet, um Erosionen, Ulcera oder Karzinome im menschlichen Verdauungstrakt zu verhüten oder zu reduzieren. Die verstärkte Schleimbildung könnte die Mucosa vor onkogenen Substanzen schützen. Zusätzlich würden onkogene Substanzen, die für den Organismus bekannt sind, durch Induktion der Verdauung unwirksam werden.

Ebenso eröffnet sich ein breites Anwendungsgebiet in der Prophylaxe bezüglich von Diarrhöeerscheinungen bei Reisenden, die auf diesen Reisen mit Nahrungsmitteln konfrontiert werden, mit denen ihr Verdauungstrakt bis dahin nicht in Berührung gekommen war. Die fehlende Induktion der Verdauung bewirkt ein Vordringen von Eiweiß in tiefere Darmabschnitte mit der daraus resultierenden möglichen Überwucherung durch Bakterien.

Auch bei der Aufzucht von Nutztieren ist es ein bekanntes Problem, daß nach Transporten der Nutztiere an die Stellen, an denen die Tiere nach den ersten Lebenswochen weiter aufgezogen werden sollen, die Milieu- und Futterveränderungen mit erheblichen Durchfällen einhergehen, die ihrerseits wieder als Ursachen für andere bakterielle und virale Erkrankungen des gesamten Gastrointestinaltrakts angesehen werden können. Eine Sensibilisierung dieser Tiere mit antigenen Substanzen, von denen bekannt ist, daß sie in dem später verabreichten Futter enthalten sind, kann diese Störungen und Krankheiten verhindern.

Eine mögliche Anwendungsweise besteht beispielsweise darin, daß Ferkel ab der dritten Lebenswoche in 14tägigen Abständen jeweils mit zwei ml einer 6%igen Molkeprotein-Lösung ohne Zusatz von Adjuvans insgesamt 4 ml parenteral sensibilisiert wurden. Nach dem Absetzen von den Muttersauen in der zweiten Woche und allmählicher Umstellung von Sauenaufzuchtfutter auf grobkörniges Mastfutter wurden die Ferkel umgestallt und gruppenweise in Laufboxen gehalten. Ab der 10.Woche wurden sämtlichen Ferkeln ausschließlich feinstgemahlenes Mastfutter angeboten. In der Literatur wird die Gabe eines derartigen Futters neben Streßfaktoren als die entscheinde Ursache für das Entstehen von Schleimhautveränderungen, wie Hyperkeratosen, Erosionen bzw. Ulcera in der gastralen Schleimhaut des Schweines angeführt.

Den Schweinen wurde während der gesamten Mastperiode bis zur Schlachtung der Schweine nach 10-wöchiger Mastdauer über die Trinkflüssigkeit zusätzlich 3 bis 6% Moleprotein angeboten.

Bei auf diese Weise behandelten Tieren traten schwere Veränderungen der Schleimhaut, wie Erosionen, weit weniger häufig auf als in Kontrollgruppen.

In analoger Weise, wie der Einsatz antigener Substanzen bei der Behandlung von Ferkeln beschrieben ist, können Kälber in den für diese Tiere zweckmäßigen Lebensabschnitten parenteral und/oder enteral sensibilisiert werden. Es werden intramuskuläre Injektionen des Antigens bei Kälbern in etwa innerhalb der ersten vier Wochen nach der Geburt verabreicht, eine zweite Injektion etwa in der sechsten Woche und eine dritte Injektion etwa in der achten bis zehnten Woche bei Umstellung der Tiere auf Kälberfutter.

In den ersten Wochen der Aufzucht erhalten die Kälber Milch und Kälberfutter und ab der achten bis zehnten Woche allgemein energiereiches Futter. Ab dem Zeitpunkt der Umstellung auf das energiereiche Futter wird vorzugsweise dem Futter und/oder dem Trinkwasser die antigene Substanz zugegeben, um das Niveau für die Protektion des Intestinaltrakts durch die beschriebenen Immunmechanismen zu induzieren und/oder zu erhalten.

Die Sensibilisierung ist auch bei adulten Wiederkäuern möglich. Bullen und Hochleistungskühe sind in zunehmendem Maße während der Periode der energiereichen Laktationsfütterung durch Magenulcera in den verschiedenen Stadien beeinträchtigt, die auch bei den erwachsenen Rindern als Ursache nachfolgender, insbesondere infektiöser Erkrankungen des gesamten Gastrointestinaltraktes angesehen werden. Die enterale oder parenterale Behandlund der Tiere mit den antigenen Substanzen kann diese Schäden verhindern.

Ein weiteres Verwendungsgebiet der antigenen Substanzen eröffnet sich in der Behandlung von Wirtschaftsgeflügel, beispielsweise Hühnern, Puten, Gänsen, Enten und Truthähnen. Als Hauptfutterquellen für die Aufzucht von Wirtschaftsgeflügel kommen Getreidekörner, Fischmehl, Soja und Erdnußschrot in Frage. Für die erwünschte Sensibilisierung im Hinblick auf die genannten Futtersubstanzen wird eine parenterale Injektion der antigenen Substanz aus praktischen Gründen am ersten Tag nach dem Eischlupf durchgeführt. Eine spätere Sensibilisierung bzw. Aufrechterhaltung einer vorangegangenen Sensibilisierung kann durch Applikation über das Trinkwasser der Tiere in den frühen Lebenstagen und -wochen erfolgen.

Die vorliegende Erfindung wird nunmehr im folgenden anhand der Figuren und von Beispielen näher erläutert.

Es zeigen:

Fig. 1      die Regulation der Antigenstimulierten Gastrinfreisetzung. Luminales Antigen wird von Antigen-präsentierenden Zellen erkannt und vermag unter anderem sowohl Mastzellen als auch T-Lymphozyten zu stimulieren. Die T-Zellprodukte Interferon-Gamma ( -IFN) und Interleukin-2 (IL-2) setzen Gastrin frei, das wiederum Histamin stimuliert. Die Mastzellprodukte Histamin, Leukotrien-$C_4$ ($LTC_4$) und Prostaglandin $E_2$ ($PGE_2$) hemmen ihrerseits die Gastrinfreisetzung (negative Rückkopplung). Der Nervus vagus kann auch auf T-Zellsysteme und Antigen-präsentierende Zellen Einfluß nehmen.

Fig. 2      Ulcus-Index im Modell des Alkoholulcus der Ratte.
Gegen NIP immunisierte Tiere zeigen nach intragastralere Applikation der Antigene einen signifikant niedrigeren Ulcusindex.

Fig. 3      Anteil an Tieren mit Läsionen, induziert nach dem SHAY-Ulcusmodell bei Kontrollen und gegen NIP-Ovalbumin immunisierten Tieren.
Die intragastrale Applikation von NIP-humanes Gamma-Globulin (NIP-HGG) führt bei immunisierten Tieren zu signifikant geringerer Ausprägung von Ulcera.

Fig. 4      Quantitative Veränderungen der Bewertungsmaßstäbe (0 und + + +) nach pathologisch-anatomischer Beurteilung der Magenschleimhaut von Schweinen im Bereich der pars proventricularis nach 10-wöchiger Fütterung feinstgemahlener Futterrationen (%).

**Beispiel 1**

Zweck des vorliegenden Beispiels war die Wirkung der immunologischen Vorgänge auf die Ulcogenese im Hinblick auf Förderung oder Protektion zu untersuchen.

Männliche Wistar-Ratten (250-350g) wurden systemisch mit NIP-OA (Nitrophenylessigsäure-Ovalbumin) immunisiert. Zur Erzeugung von Ulcera wurde in Äthernarkose 1ml absoluter Alkohol intragastral verabreicht. 15 Min. vor der Alkoholapplikation hatte eine Gruppe (I) der Tiere (n = 12) das Atigen NIP (gebunden an HGG als Trägerprotein) erhalten, Gruppe II (n = 12) das Trägerprotein HGG allein. Als weitere Kontrollen dienten nicht immunisierte Tiere: 6 hatten NIP-HGG (III), weitere 6 HGG (IV) verabreicht bekommen. 1 Stunde nach der Alkoholgabe erfolgte die Beurteilung der Läsionen an den entnommenen Mägen makroskopisch durch Messung der Länge hämorrhagischer Läsionen.

Systemisch NIP-immunisierte Tiere entwickeln nach oraler NIP-Applikation und nachfolgender Alkoholgabe signifikant (p = 0,02) weniger Schleimhautläsionen als alle Kontrollgruppen.

## Tabelle 1

| | | Immunisierung | Intragstrale Gabe von | Länge hämorrhabischer Läsionen | |
|---|---|---|---|---|---|
| I | (n=12) | NIP | NIP | 24 | p=0,02 |
| II | (n=12) | NIP | HGG | 57 | |
| III | (n=6) | nicht immunis. | NIP | 72 | |
| IV | (n=6) | nicht immunis. | HGG | 78 | |

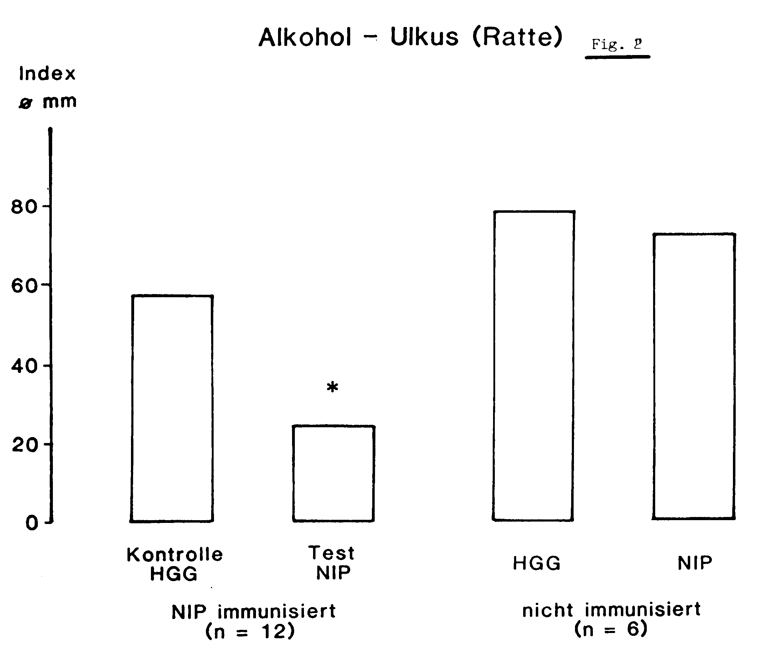

Die intraluminale antigenspezifische Stimulierung des Magens immunisierter Wistar-Ratten vermindert signifikant die Entstehung von Schleimhautläsionen. Damit kann erstmals eine immunologisch vermittelte Ulcoprotektion gezeigt werden (Fig. 2).

**Beispiel 2**

Die Immunisierung von männlichen Wistar-Ratten (250-350g) erfolgte mit NIP-OA (Nitrophenylessigsäure-Ovalbumin). Als Ulcusmodell diente der am Pylorus ligierte Magen (SHAY-Ulcus). Immunisierten Ratten wurde nach Ligatur des Pylorus in Chloralhydratnarkose das Antigen NIP (gekoppelt an HGG = humanes Gamma-Globulin) intragastral injiziert (Testgruppe). Die Kontrolltiere erhielten lediglich das Trägerprotein HGG: Eine weitere Kontrollgruppe bestand in scheinoperierten Tieren (ohne Pylorusligatur). 18 Stunden nach der Operation erfolgte die Beurteilung der Magenwandveränderungen makroskopisch und mikroskopisch. Als Läsionen wurden definiert: Perforationen, konfluierende transmurale Ulcera und Wandnekrosen > 0,5 cm Durchmesser.

Tiere, die systemisch NIP-spezifisch immunisiert waren, entwickelten nach Pylorusligatur und intragastraler Applikation von NIP-HGG signifikant (p < 0,05) weniger Läsionen als die Kontrolltiere, die HGG erhalten hatten. Scheinoperierte Ratten wiesen keinerlei Schleimhautläsionen auf.

Tabelle 2

| NIP-spezifisch immunisierte Tiere | | Intragastrale Applikation von | Anteil mit Läsionen |
|---|---|---|---|
| Testgruppe | (n=24) | NIP-HGG | 7/24 |
| Kontrollgruppe | (n=23) | HGG | 14/23 |
| Scheinoperierte | (n=6 bzw.6) | NIP-HGG bzw.HGG | 0/6 bzw. 0/6 |

Die antigenspezfische Stimulation von gastralen Funktionen führt in einem definierten Ulcusmodell (Pylorusligatur) zur signifikanten Verminderung von Wandläsionen (Fig. 3).

**Beispiel 3**

**Durchführung der Immunisierung von Jungschweinen**

Unter der Voraussetzung, daß die Antikörperproduktion beim Ferkel ab der 2. Woche beginnt und unter Berücksichtigung der Praktikabilität werden Jungschweine geimpft, wenn sie während der Aufzucht vom Züchter Eiseninjektionen erhalten.

Als Antigen könnte jedes biologische Protein oder eine synthetische antigene, immunstimulierende Substanz verwendet werden, die nach der Aufnahme durch das Jungschwein rückstandsfrei abgebaut wird oder nur in geringen Mengen als solche augeschieden wird.

Für die vorliegende Immunisierung der Ferkel wurde Molkeprotein, und zwar als 6%ige Molkeprotein-Lösung, ohne Zusatz von Adjuvans bzw. im späteren Verlauf der Immunisierung als 3 - 6%iger Zusatz in Trinkflüssigkeit verabreicht.

Für eine Untersuchung der prophylaktischen Wirkung der als Antigene verabreichten Molkeproteine auf die gastralen Schleimhautveränderungen wurden 30 Schweine als Testgruppe und 30 Schweine als Kontrolltiere eingeteilt. Während des Versuches starben als Ferkel, bzw.bei der Umstellung, fünf Schweine der Testgruppe und zwei der Kontrolltiere.

Die Versuchstiere wurden nach folgendem Schema vorbehandelt: Ferkel wurden ab der dritten Lebenswoche in 14-tägigen Abständen jeweils mit 2 ml einer 6%igen Molkeprotein-Lösung ohne Zusatz von Adjuvans insgesamt 4 ml parenteral sensibilisiert.

Nach Absetzen von den Muttersauen in der zweiten Woche und allmählicher Umstellung von Sauenaufzuchtfutter auf grobkörniges Mastfutter wurden sowohl die Versuchs- als auch die Kontrolltiere umgestallt und gruppenweise in Laufboxen gehalten.

Ab diesem Zeitpunkt, etwa der 10. Woche, wurde sämtlichen Schweinen ausschließlich feinstgemahlenes Mastfutter angeboten. In der Literatur wird die Gabe eines derartigen Futters neben Streßfaktoren als die entscheidende Ursache für das Entstehen von Schleimhautveränderungen, wie Hyperkeratosen, Erosionen bzw. Ulcera in der gastralen Schleimhaut des Schweines angeführt.

Beiden Tiergruppen wurde während der gesamten Mastperiode bis zum Schlachten der Schweine nach 10-wöchiger Mastdauer über die Trinkflüssigkeit zusäützlich 3-6% Molkeprotein angeboten.

Die Änderungen der Magenschleimhaut von Schweinen im Bereich der pars proventricularis wurde nach pathologisch-anatomischen Kriterien beurteilt.

Tabelle 3 zeigt die Häufigkeit der Bewertungsmaßstäbe (0 bis + + +) nach 10-wöchiger Fütterung feinstgemahlener Futterrationen und deren prozentuale Anteile (%).

## Tabelle 3

| 0 | + | + + | + + + | |
|---|---|---|---|---|
| 6 21,4% | 6 21,4% | 8 28,6% | 8 28,6% | Kontrollgruppe n = 28 |
| 10 40% | 7 28% | 6 24% | 2 8% | Versuchsgruppe n = 25 |

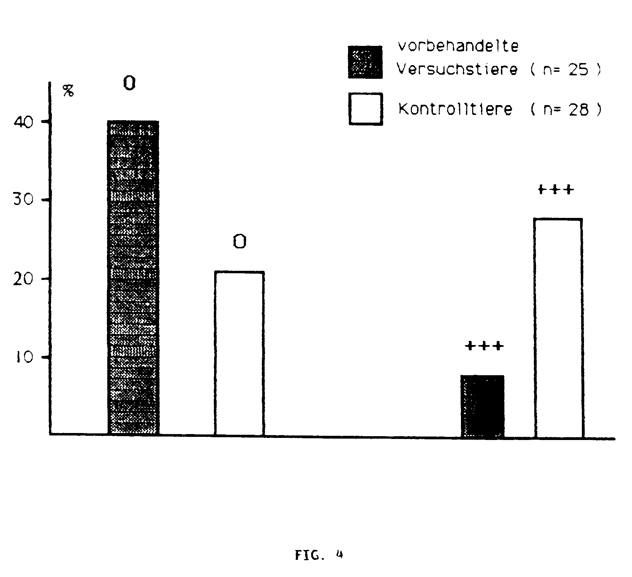

In Fig. 4 ist graphisch dargestellt, daß bei den mit Molkeprotein behandelten Tieren doppelt so viele Individuen eine intakte Schleimhaut aufwiesen als bei den Kontrolltieren (die beiden linken Säulen in Fig. 4 mit der Bezeichnung "0", das für intakte Schleimhaut verwendet wird). Die beiden rechten Säulen der Fig. 4 hingegen lassen erkennen, daß schwere Veränderungen der Schleimhaut, beispielsweise Erosionen, die mit "+ + +" bezeichnet sind, um 70% weniger häufig auftraten als in der Kontrollgruppe.

Die exakten Zahlen der makroskopischen Auswertung der Magenschleimhäute der Versuchstiere, wie sie aus Tabelle 3 ersichtlich sind, ergeben, daß in Versuchsmodellen geringgradige, flache Hyperkeratosen (mit "+") bzw. mittelgradige, spitzzackige Hyperkeratosen (mit "+ +") bezeichnet, in beiden Gruppen gleich häufig auftraten. Entscheidend sind jedoch die Veränderungen "+ + +", d.h. die Erosionen, die sich in diesem Versuch beim Schwein noch deutlicher haben verhindern lassen als in den in den Beispielen 1 und 2 geschilderten Experimenten.

**Beispiel 4**

**Durchführung der Immunisierung von Rindern**

Als Antigen wird ein Protein verwendet, das in dem Kälberfutter, insbesondere dem Milchaustauscher, mit dem die Kälber nach der Geburt gefüttert werden, nicht enthalten ist, jedoch in dem energiereichen Futter, auf das die Kälber etwa in der achten bis zehnten Woche nach der Geburt umgestellt werden, vorkommt. Die ulcerösen Veränderungen bei den Kälbern treten bevorzugt während der Umstellung auf das energiereiche Futter, in der Regel im Alter von vier bis zehn Wochen, auf. Diese Störungen stehen im Zusammenhang mit der geschilderten, fehlenden Sensibilisierung der Tiere bezüglich der in dem energiereichen Futter enthaltenen Proteine. Hauptbestandteile des energiereichen Futters für Wiederkäuer sind vornehmlich Mais, Weizen, Soja, Gerste, Grassilage und Heu, das sich entsprechend dem Grad der Intensivmast in seiner Zusammensetzung unterscheidet.

Die erste intramuskuläre Injektion der verwendeten antigenen Substanz wird beim Kalb innerhalb der ersten vier Wochen nach der Geburt vorgenommen. Eine zweite Injektion erfolgt in der sechsten Woche und eine dritte Injektion sodann bei Umstellung auf energiereiches Kälberfutter in der achten bis zehnten Lebenswoche. In der ersten Woche der Aufzucht erhalten die Kälber Milch und "Starterfutter". Ab etwa der achten bis zehnten Woche sodann energiereiches Futter. Ab dem Zeitpunkt der Umstellung auf energiereiches Futter wird dem Futter und/oder dem Trinkwasser die antigene Substanz zugegeben, um die immunologische Protektion des Gastrointestinaltrakts durch den beschriebenen Immunmechanismus zu induzieren oder etablieren.

Die Dosierung der antigenen Substanz beträgt bis zu 0,1 g Testsubstanz/ kg Körpergewicht bei intramuskulärer Applikation und wird zwei- bis dreimal wiederholt.

Für die Intensivierung der protektiven Mechanismen wird die antigene Substanz sodann dem Trinkwasser in einer Menge von bis zu 1g/100ml Wasser zugegeben.

Durch die geschilderte Behandlung werden die ulcerösen Veränderungen im Gastrointestinaltrakt von Kälbern verhindert oder reduziert.

Nach der geschilderten Behandlung der Rinder sind diese gegenüber jeder Änderung des Stallklimas, Futterwechsels, Transports ("Crowding Disease"), insbesondere in den ersten Wochen nach der Geburt sehr viel besser geschützt. Die mit den genannten Milieuveränderungen häufig auftretenden Störungen oder Erkrankungen des Gastrointestinaltrakts, insbesondere der Durchfälle, die wiederum als Ursachen für weitere bakterielle und virale Erkrankungen des gesamten Verdauungstraktes angesehen werden können, können auf diese Weise verhindert werden.

Im Bereich der adulten Wiederkäuer findet die Verwendung von antigenen Substanzen in der Weise Anwendung, daß Bullen und Hochleistungsmilchkühe während der Periode der intensiven und energiereichen Laktationsfütterung die erforderlichen antigenen Substanzen appliziert werden. Auf diese Weise können auch beim ausgewachsenen Tier die immer häufiger auftretenden Magenulcera in den verschiedenen Stadien verhindert oder reduziert werden.

**Beispiel 5**

**Durchführung der Immunisierung von Geflügel**

Analog den Immunisierungen von Schweinen und Rindern wird Wirtschaftsgeflügel, beispielsweise Hühner, Puten, Gänse, Enten oder Truthähne in der geschilderten Weise mit den antigenen Substanzen sensibilisiert. Die antigenen Substanzen werden gemäß den Hauptfutterquellen der späteren Aufzucht solche aus Getreidekörnern, Fischmehl, Soja oder Erdnußschrot sein.

Die parenterale Injektion der antigenen Substanz erfolgt aus praktischen Gründen am ersten Tag nach dem Eischlupf. Diese erste Sensibilisierung wird sodann durch spätere Applikation der antigenen Substanz intensiviert und etabliert, wobei die Verabreichung der antigenen Substanz in diesem Stadium in enteraler Form etwa eine Woche nach dem Schlüpfen über das Trinkwasser erfolgt.

**Patentansprüche**

1. Verwendung von mindestens einer antigenen Substanz zur Herstellung eines Medikamentes zur Prophylaxe und Therapie von Störungen und Krankheiten im Verdauungtrakt von Tieren und Menschen, wobei nach vorrausgegangener Immunisierung bzw. Sensibilisierung durch die antigene(n) Substanz(en) eine nachfolgende gastrointestinale Applikation der antigenen Substanz(en) in geeigneter Formulierung erfolgt, mit folgenden Ausnahmen:
(a) ein Verfahren zur Verhinderung von Diarrhöe als Folge einer Kokzidieninfektion bei Geflügel durch Aufzucht mit einem Futter, das zugesetzte, lebensfähige, sporulierte Oozysten wenigstens einer Kokzidienart enthält, für die das Geflügel anfällig ist, wobei die Oozysten in einer Konzentration vorhanden sind, die nur ausreichend ist, um eine subklinische Infektion im Geflügel zu induzieren;
(b) ein Verfahren zur Prophylaxe von Darmkrankheiten bei Schweinen durch orale Immunisierung und weitere orale Gabe von hitzeinaktivierten Antigen-Zubereitungen von E.coli-Stämmen, welche zu Darmkrankheiten führen; und
(c) ein Verfahren zur Prophylaxe und Therapie von Darmkrankheiten bei präruminanten Kälbern durch orale Immunisierung und weitere orale Gabe von hitze-inaktivierten Antigen-Zubereitungen von E. coli- und Salmonella-Stämmen, welche zu Darmkrankheiten führen.

2. Verwendung von mindestens einer antigenen Substanz nach Anspruch 1, wobei die antigene Substanz pflanzliches Eiweiß, vorzugsweise aus Mais oder Soja, ist.

3. Verwendung von mindestens einer antigenen Substanz nach Anspruch 1, wobei die antigene Substanz tierisches Eiweiß ist.

4. Verwendung von mindestens einer antigenen Substanz nach Anspruch 3, wobei das tierische Eiweiß Molkeprotein ist.

5. Verwendung von mindestens einer antigenen Substanz nach Anspruch 4, wobei das Molkeprotein als 3-9, vorzugsweise 6%ige Molkeprotein-Lösung verwendet wird.

6. Verwendung von mindestens einer antigenen Substanz nach Anspruch 1, wobei die antigene Substanz menschliches Eiweiß, vorzugsweise aus Blut oder Blutbestandteilen, nämlich Serum, Plasma oder Hämoglobin, ist.

7. Verwendung von mindestens einer antigenen Substanz nach Anspruch 1, wobei die antigene Substanz virales Eiweiß ist.

8. Verwendung von mindestens einer antigenen Substanz nach Anspruch 1, wobei die antigene Substanz ein Mikroorganismus oder ein Bestandteil eines Mikroorganismus ist.

EP 0 286 847 B1

**9.** Verwendung von mindestens einer antigenen Substanz nach Anspruch 8, wobei die antigene Substanz ein Bakterium oder ein Zellwandbestandteil eines Bakteriums ist.

**10.** Verwendung von mindestens einer antigenen Substanz nach Anspruch 8, wobei der Mikroorganismus ein Vertreter der Protozoen ist.

**11.** Verwendung von mindestens einer antigenen Substanz nach Anspruch 1, wobei die antigene Substanz ein synthetisches Protein, vorzugsweise ein kurzes Peptid ist, das antigene Determinanten für das Auslösen der Immunantwort enthält.

**12.** Verwendung von mindestens einer antigenen Substanz nach Anspruch 1, wobei die antigene Substanz ein Hapten, vorzugsweise Nitrophenylessigsäure (NIP), ist.

**13.** Verwendung von mindestens einer antigenen Substanz nach Anspruch 12, wobei das Hapten an Trägerproteine gekoppelt ist.

**14.** Verwendung von mindestens einer antigenen Substanz nach einem der Ansprüche 1 bis 13, wobei zusätzlich zu der antigenen Substanz Immunmodulatoren verwendet werden.

**15.** Verwendung von mindestens einer antigenen Substanz nach einem der vorhergehenden Ansprüche, wobei die antigene Substanz für die enterale Applikation, vorzugsweise als Aerosol, konfektioniert ist, gegebenenfalls unter Zusatz von pharmazeutisch verträglichen Hilfsstoffen.

**16.** Verwendung von mindestens einer antigenen Substanz nach einem der Ansprüche 1 bis 14, wobei die antigene Substanz zur parenteralen Applikation, vorzugsweise intramuskulär und/oder subkutan, konfektioniert ist, gegebenenfalls unter Zusatz von pharmazeutisch verträglichen Hilfsstoffen.

**17.** Verwendung von mindestens einer antigenen Substanz nach Anspruch 15 oder 16 zur Prophylaxe oder Therapie von Erosionen, Ulcera oder Karzinomen im menschlichen Verdauungstrakt.

**18.** Verwendung von mindestens einer antigenen Substanz nach Anspruch 15 oder 16, zur Prophylaxe von Diarrhöe, verursacht durch Nahrungs- oder Futtermittel, mit denen der menschliche oder tierische Verdauungstrakt noch nicht in Berührung gekommen ist.

## Claims

**1.** Use of at least one antigenic substance for the preparation of a medicament for the prophylaxis and treatment of disorders and diseases in the digestive tract of animals and humans, in which method, after prior immunization or sensitization by the antigenic substance(s), a subsequent gastrointestinal administration of the antigenic substance(s) in a suitable formulation is carried out, with the following exceptions:

(a) a method for preventing diarrhoea in poultry, as a consequence of a coccidial infection, by rearing them with a feed which contains added, live, sporulated oocysts of at least one coccidia species to which the poultry is susceptible, the oocysts being present in a concentration which is sufficient only to induce a subclinical infection in the poultry;

(b) a method for the prophylaxis of intestinal diseases in pigs by oral immunization and additional oral administration of heat-inactivated antigen formulations of E. coli strains, which lead to intestinal diseases; and

(c) a method for the prophylaxis and treatment of intestinal diseases in pre-ruminant calves by oral immunization and additional oral administration of heat-inactivated antigen formulations of E. coli and Salmonella strains, which lead to intestinal diseases.

**2.** Use of at least one antigenic substance according to Claim 1, in which the antigenic substance is vegetable protein, preferably from maize or soya.

**3.** Use of at least one antigenic substance according to Claim 1, in which the antigenic substance is animal protein.

12

EP 0 286 847 B1

4. Use of at least one antigenic substance according to Claim 3, in which the animal protein is whey protein.

5. Use of at least one antigenic substance according to Claim 4, in which the whey protein is used as a 3-9% strength, preferably 6% strength, whey protein solution.

6. Use of at least one antigenic substance according to Claim 1, in which the antigenic substance is human protein, preferably from blood or blood constituents, namely serum, plasma or haemoglobin.

7. Use of at least one antigenic substance according to Claim 1, in which the antigenic substance is viral protein.

8. Use of at least one antigenic substance according to Claim 1, in which the antigenic substance is a microorganism or a constituent of a microorganism.

9. Use of at least one antigenic substance according to Claim 8, in which the antigenic substance is a bacterium or a component of the cell wall of a bacterium.

10. Use of at least one antigenic substance according to Claim 8, in which the microorganism is a representative of the protozoa.

11. Use of at least one antigenic substance according to Claim 1, in which the antigenic substance is a synthetic protein, preferably a short peptide, which contains antigenic determinants for triggering the immune response.

12. Use of at least one antigenic substance according to Claim 1, in which the antigenic substance is a hapten, preferably nitrophenylacetic acid (NIP).

13. Use of at least one antigenic substance according to Claim 12, in which the hapten is coupled to carrier proteins.

14. Use of at least one antigenic substance according to one of Claims 1 to 13, in which immune modulators are used in addition to the antigenic substance.

15. Use of at least one antigenic substance according to one of the preceding claims, in which the antigenic substance is produced for administration by the intestinal route, preferably as an aerosol, if appropriate with the addition of pharmaceutically tolerated auxiliaries.

16. Use of at least one antigenic substance according to one of Claims 1 to 14, in which the antigenic substance is produced for parenteral administration, preferably intramuscular and/or subcutaneous administration, if appropriate with the addition of pharmaceutically tolerated auxiliaries.

17. Use of at least one antigenic substance according to Claim 15 or 16, for the prophylaxis or treatment of erosions, ulcers or carcinomas in the human digestive tract.

18. Use of at least one antigenic substance according to Claim 15 or 16, for the prophylaxis of diarrhoea caused by foodstuffs or feedstuffs with which the human or animal digestive tract has not yet come into contact.

**Revendications**

1. Application d'au moins une substance antigène à la préparation d'un médicament pour la prophylaxie et la traitement des désordres et des maladies de l'appareil digestif des animaux et de l'homme, dans lequel après immunisation au sensibilisation préalable par la ou les substance(s) antigène(s) on procède à une application gastro-intestinale ultérieure de la ou des substance(s) antigène(s) dans une formulation appropriée, avec les exceptions suivantes:
    (a) un procédé pour inhiber la diarrhée à la suite d'une infection par une coccidie chez les volailles par élevage avec une nourriture qui contient des oocystes sporulés vivants ajoutés appartenant à au moins un type de coccidie, auxquels la volaille est sensible, oû les oocystes sont présents à une concentration qui suffit simplement pour induire une infection sub-clinique chez la volaille;

(b) un procédé de prophylaxie des maladies intestinales chez les porcs par immunisation orale et administration orale ultérieure du préparations d'antigènes de souches d'E. coli qui conduisent à des maladies intestinales inactivées à la chaleur; et

(c) un procédé de prophylaxie et de traitement des maladies intestinales chez les veaux pré-ruminants par immunisation orale et administration orale ultérieure de préparations d'antigènes de souches d'E. coli et de Salmonella qui conduisent à des maladies intestinales inactivèes à la chaleur.

2. Application d'au moins une substance antigène selon la revendication 1, oû la substance antigène est une protéine végétale, de préférence de mais ou de soja.

3. Application d'au moins une substance antigène selon la revendication 1, dans laquelle la substance antigène est une protéine animale.

4. Application d'au moins une substance antigène selon la revendication 3, dans laquelle la protéine animale est une protéine de petit-lait.

5. Application d'au moins une substance antigène selon la revendication 4, dans laquelle la protéine de petit-lait est utilisée sous la forme d'une solution de protéine de petit-lait à 3-9, de préférence à 6%.

6. Application d'au moins une substance antigène selon la revendication 1, dans laquelle la substance antigène est une protéine humaine, de préférence provenant du sang ou des constituants du sang, à savoir le sérum, le plasma ou l'hémoglobine.

7. Application d'au moins une substance antigène selon la revendication 1, dans laquelle la substance antigène est une protéine virale.

8. Application d'au moins une substance antigène selon la revendication 1, dans laquelle la substance antigène est un microorganisme ou un constituant d'un microorganisme.

9. Application d'au moins une substance antigène selon la revendication 8, dans laquelle la substance antigène est une bactérie ou un constituant de la paroi cellulaire d'une bactérie.

10. Application d'au moins une substance antigène selon la revendication 8, dans laquelle le microorganisme est un représentent des protozoaires.

11. Application d'au moins une substance antigène selon la revendication 1, dans laquelle la substance antigène est une protéine de synthése, de préférence un peptide court, qui contient des déterminants antigéniques pour le déclenchement de la réponse immunitaire.

12. Application d'au moins une substance antigène selon la revendication 1, dans laquelle la substance antigène est un haptène, de préférence l'acide nitrophénylacétique (NIP).

13. Application d'au moins une substance antigène selon la revendication 12, dans laquelle l'haptène est couplé à une protéine support.

14. Application d'au moins une substance antigène selon l'une des revendication 1 à 13, dans laquelle on utilise, outre la substance antigène, des immunomodulateurs.

15. Application d'au moins une substance antigène selon l'une des revendications précédentes, dans laquelle la substance antigène est confectionnée en vue de l'application entérale, de préférence sous forme d'aérosol, le cas échéant avec addition d'adjuvants pharmaceutiquement acceptables.

16. Application d'une substance antigène selon l'une des revendications 1 à 14, dans laquelle la substance antigène est confectionnée en vue de l'application parentérale, de préférence intramusculaire et/ou sous-cutanée, la cas échéant avec addition d'adjuvants pharmaceutiquement acceptables.

17. Application d'au moins une substance antigène selon la revendication 15 ou 16 à la prophylaxie ou au traitement des érosions, des ulcères ou des carcinomes dans l'appareil digestif humain.

18. Application d'au moins une substance antigène selon la revendication 15 ou 16, à la prophylaxie de la diarrhée provoquée par des aliments ou du fourrage avec lesquels l'appareil digestif humain ou animal n'est pas encore entré en contact.

Fig. 1

N. VAGUS

Gastrin ⊕

Mastzelle

Gastrin

G-Zelle

Histamin ⊖

LTC$_4$ ⊖

PGE$_2$ ⊖

IL-2

γ-IFN

T-Lymphozyten

+/−

Klasse II−Antigen

+/−

„Antigenpräsentierende Zelle"

ANTIGEN

Regulation der antigenstimulierten Gastrinfreisetzung

# Alkohol – Ulkus (Ratte)  <u>Fig. 2</u>

Index
ø mm

EP 0 286 847 B1

Anteil mit
Läsionen
%

Shay – Ulkus (Ratte)    Fig. 3

80

60

40

20

0

*

Kontrolle

Test
(immunisiert)

(n = 23)

(n = 24)

EP 0 286 847 B1

FIG. 4